# EUROPÄISCHE PATENTSCHRIFT

(11) **EP 3 763 222 B1**
(45) Veröffentlichungstag und Bekanntmachung des Hinweises auf die Patenterteilung: **02.04.2025**
(21) Anmeldenummer: 20183458.7
(22) Anmeldetag: 01.07.2020
(51) Int. Cl.: A23K 10/38, A23J 1/00, A23J 1/12, A23J 1/16, A23K 20/147

(54) **VERFAHREN ZUR GEWINNUNG EINER KONZENTRIERTEN PROTEINREICHEN PHASE AUS RESTSTOFFEN DER BIOETHANOLPRODUKTION**
METHOD FOR OBTAINING A CONCENTRATED PROTEIN RICH PHASE FROM RESIDUES OF BIOETHANOL PRODUCTION
PROCÉDÉ D'EXTRACTION D'UNE PHASE CONCENTRÉE RICHE EN PROTÉINES DES RÉSIDUS DE FABRICATION DE BIOÉTHANOL

(30) Priorität: 03.07.2019 DE 102019004704
(43) Veröffentlichungstag der Anmeldung: 13.01.2021
(73) Patentinhaber: Verbio SE, 06780 Zörbig (DE)
(72) Erfinder: Pohl, Julia, 06844 Dessau-Roßlau (DE); Kühling, Jan, 06114 Halle (Saale) (DE); Schlimbach, Michael, 06120 Halle/Saale (DE); Klein, Wolfram, 04205 Leipzig (DE); Lüdtke, Oliver, 04416 Markkleeberg (DE); Lamp, Anne, 20357 Hamburg (DE)
(74) Vertreter: Gulde & Partner

(56) Entgegenhaltungen:
- EP-A1- 2 481 293
- EP-A1- 2 699 655
- WO-A2-02/38787
- US-A1- 2015 305 370
- US-A1- 2019 119 711

## Beschreibung

### Definitionen:

Der Begriff "Anmaischung" beschreibt den Vorgang, bei dem Getreidemehl mit Flüssigkeit versetzt wird, wobei die Flüssigkeit ein Gemisch aus Prozessflüssigkeiten und ggf. Wasser ist.

Der Begriff "Dünnschlempe" umfasst in dieser Erfindung die flüssige Phase, die durch eine Fest-Flüssig-Trennung der Schlempe erzeugt wird.

Unter "Gelöst-TS-Gehalt" ist im Rahmen dieser Erfindung die Trockensubstanz zu verstehen, die nach Zentrifugation und Feinfiltration (Porengröße 0,2 µm) einer verdünnten Ausgangsprobe, wie z.B. Schlempe, in gelöster Form im Filtrat vorliegt. Der Gelöst-TS-Gehalt wird in Ma% angegeben.

Unter "Kaltmaischverfahren" ist der drucklose enzymatische Aufschluss des eingemaischten Getreidemehls bei einer Temperatur unterhalb der Verkleisterungstemperatur der Stärke zu verstehen, vorzugsweise im Bereich zwischen 45°C und 66°C. Dabei werden die Stärkemoleküle sowie die Stärke-umhüllenden Proteine aufgebrochen und teilweise erfolgt eine Verzuckerung der Stärke.

Der Begriff "Klarphase" bezeichnet in dieser Erfindung die wässrige Phase, die bei der Trennung der verdünnten Dünnschlempe entsteht.

Der Begriff "Konzentrat" bezeichnet die proteinreiche Phase, die aus der verdünnten Dünnschlempe erzeugt wird.

Der Begriff "Prozessflüssigkeit" umfasst in dieser Erfindung alle feststoffhaltigen sowie alle feststofffreien Flüssigkeiten, die im Prozess anfallen.

Unter "Rohproteingehalt" ist in dieser Erfindung der Anteil des Rohproteins an der Trockensubstanz zu verstehen. Er wird in %TS (w/w) angegeben.

Der Begriff "Schlempe" umfasst in dieser Erfindung den Rückstand aus der Destillation einer ethanolhaltigen Getreidemaische.

Der Begriff "Treber" steht innerhalb dieser Erfindung für die feste Phase, die durch eine Fest-Flüssig-Trennung aus der Schlempe abgetrennt wird.

Die "Trennkorngröße" beschreibt die Partikelgröße, die nach der Separation einer Ausgangsprobe mit verschiedenen Partikelgrößenfraktionen, wie z.B. Schlempe, jeweils zu 50% in der groben Phase, wie z.B. Treber, und in der feinen Phase, wie z.B. Dünnschlempe, vorliegt.

Der Begriff "Zulauf" bezeichnet die verdünnte Dünnschlempe, die in Klarphase und Konzentrat aufgetrennt wird.

### STAND DER TECHNIK

Die Produktion von Bioethanol aus pflanzlichen Rohstoffen ist bekannt. In Deutschland hat sich die Menge des hergestellten Bioethanols in den letzten 10 Jahren mehr als verdoppelt. Der Trend für die Zukunft ist positiv, da in den nächsten Jahren eine weitere Reduktion des CO₂-Ausstoßes von Kraftstoffen gesetzlich vorgeschrieben ist, die durch die Beimischung von Bioethanol erzielt werden kann. Weltweit betrachtet, stellen die kleinkörnigen Getreidearten, wie z.B. Roggen, Weizen und Triticale, neben Mais einen der Hauptrohstoffe für die Bioethanolproduktion dar.

Für die Bioethanolproduktion wird das Getreide vermahlen, angemaischt und unter Zusatz von Hefe fermentiert. Die fermentierte Maische wird einer Destillation zugeführt, bei der durch die Abtrennung des Ethanols die sogenannte Schlempe entsteht, die sich aus nicht zu Ethanol umgesetzten Komponenten, Salzen und Hefe zusammensetzt. In der Regel wird die Schlempe einer Fest-Flüssig-Trennung unterzogen, bei der die festen Getreiderückstände von der flüssigen Phase, der Dünnschlempe, abgetrennt werden. Die abgetrennten Feststoffe werden häufig als Treber bezeichnet. Dünnschlempe und Treber werden in verschiedenen Varianten einzeln oder in Kombination vorwiegend als Futtermittel verwendet.

Ein bekanntes und vor allem in den USA weit verbreitetes Futtermittel ist das sogenannte DDGS (Dried Distillers Grains with Solubles), welches aus getrocknetem Treber und getrocknetem Dünnschlempe-Konzentrat besteht und einen Trockensubstanzgehalt von ca. 90% aufweist. Durch die Trocknung werden die enthaltenen Nährstoffe im Vergleich zur flüssigen Schlempe aufkonzentriert, wodurch ein höherwertiges Futtermittel entsteht. Das bei der Trocknung bzw. Eindampfung anfallende Kondensat wird in die Anmaischung zurückgeführt, sodass der Frischwasserbedarf reduziert bzw. vollständig gedeckt wird.

Der Rohproteingehalt im DDGS beträgt in etwa 30%TS, d.h. bei einem TS-Gehalt von 90% macht das Rohprotein anteilig 27% der Originalsubstanz aus. Im Vergleich dazu enthält flüssige Schlempe nur ca. 2-4% Rohprotein bezogen auf die Originalsubstanz. Aufgrund des hohen Fasergehaltes im DDGS kann dieses Futtermittel jedoch nur an Wiederkäuer verfüttert werden.

Der Rohproteingehalt ist ein wesentliches Kriterium bei der Bewertung eines Futtermittels. Je höher der Rohproteingehalt, umso wertvoller ist das Futtermittel. Aus diesem Grund wird eine Erhöhung des Rohproteingehaltes angestrebt, um ein Futtermittel aufzuwerten. Gleichzeitig soll der Fasergehalt minimal sein, damit die Anwendung des Futtermittels nicht auf Wiederkäuer beschränkt ist.

Verschiedene Verfahren zur Abtrennung von Proteinen aus Schlempe bzw. zur Anreicherung der Proteine in einzelnen Schlempefraktionen sind dem Fachmann bekannt.

Eine mögliche Variante zur Gewinnung von Proteinen aus Schlempe besteht in der Extraktion dieser. EP2874503 A1 beschreibt ein Verfahren zur Extraktion von Proteinen aus Treber mit Ethanol. Dazu wird der Treber volumetrisch mit 50% Ethanol verdünnt. Nach einer Verweilzeit von ca. 20 min bei ca. 49°C wird der Feststoff vom proteinhaltigen Lösungsmittel abgetrennt. Zur Isolierung der Proteine muss das Lösungsmittel anschließend noch entfernt werden.

Dieses Verfahren ist zum einen nachteilig, da nur der geringe Anteil an Proteinen abgetrennt wird, der im Treber enthalten ist und die Proteine in der Dünnschlempe nicht berücksichtigt werden. Zum anderen wirken sich der hohe Bedarf an Ethanol und der zusätzliche Schritt zur Entfernung des Ethanols nachteilig auf die Kosten des Prozesses aus.

Ein weiteres Verfahren, bei dem die Proteinabtrennung unter Zusatz einer Chemikalie erfolgt, ist Teil des im Patent US9516891 B1 beschriebenen komplexen Prozesses zur Aufarbeitung von Schlempe. Dabei wird Dünnschlempe mit kationischen und anionischen Copolymeren, wie z.B. Acrylamid-Acrylsäure-Copolymeren versetzt, die eine Ausflockung der Proteine bewirken. Die ausgeflockten Proteine lassen sich im weiteren Verlauf mithilfe eines Trikanters abtrennen.

Als Nachteile sind hier die Kosten für das Flockungshilfsmittel sowieso die eingeschränkte Verwendbarkeit des Proteinproduktes aufgrund des enthaltenen Flockungshilfsmittels zu sehen. Aus dem Patent geht nicht hervor, ob und wie das Flockungshilfsmittel wieder von den Proteinen abgetrennt wird und demzufolge kann das Produkt nicht ohne weitere Behandlung als Futtermittel verwendet werden.

Verfahren zur Proteinabtrennung aus Schlempe, die ohne Zusatz von Chemikalien und Flockungshilfsmitteln auskommen, sind in Bezug auf die Anwendbarkeit des Produktes als Futtermittel am besten geeignet. Das Proteinprodukt enthält in diesem Fall keine chemischen Verunreinigungen, ebenso ist kein zusätzlicher Prozessschritt zur Abtrennung der Chemikalien erforderlich.

Das in WO2016019374 A2 beschriebene Verfahren beinhaltet eine Temperaturbehandlung der Schlempe im Bereich von 93°C bis 177°C über eine Dauer von 3 min bis 180 min. Durch diese Temperaturbehandlung denaturieren die Proteine und bilden Agglomerate aus, die sich besser abtrennen lassen, beispielsweise durch Schwerkraftsedimentation oder auch Zentrifugation.

Als wesentlicher Nachteil ist hier der hohe Energiedarf für die Temperaturbehandlung der Schlempe zu betrachten.

EP2481293 B1 offenbart ein temperaturschonendes Verfahren zur Erzeugung eines proteinhaltigen Produktes aus Dünnschlempe (TS-Gehalt ca. 4%), bei dem die Dünnschlempe in einer Filtrationsanlage, z.B. einer Ultrafiltration, aufkonzentriert wird. Ein Teilstrom des Retentats aus der Filtrationsanlage wird dabei in den Vorlagebehälter für die Filtration zurückgeführt, um für den optimalen Betriebspunkt der Filtrationsanlage einen TS-Gehalt von ca. 7% einzustellen. Optional kann sich am Ende noch eine temperaturschonende Trocknung und Pelletierung des Proteinproduktes anschließen.

Der hohe apparative und operative Aufwand aufgrund der Filtrationsanlage ist hier als Nachteil zu bewerten.

Dem Fachmann ist bekannt, dass Tellerzentrifugen häufig bei der Abtrennung von Proteinen aus Dünnschlempe eingesetzt werden. Entsprechende Beispiele sind unter anderem in EP2699655 A1 und EP2741615 A2 zu finden.

Soll nicht die gesamte Dünnschlempe zu getrockneten Futtermitteln verarbeitet werden bzw. sieht das Anlagenkonzept generell keine Trocknung der Dünnschlempe vor, kann durch eine Rückführung mindestens eines Teils der Dünnschlempe in die Anmaischung der Bedarf an Frischwasser auf diese Weise reduziert werden. Der rückführbare Anteil ist jedoch nach bisherigem Stand aufgrund mehrerer Faktoren begrenzt oder es bedarf einer speziellen Behandlung.

In EP2864460 A1 ist beschrieben, dass bei einer Rückführung der Dünnschlempe die enthaltenen Proteine und Nährstoffe zwar einen positiven Effekt auf die Fermentation haben, den weitaus größeren negativen Aspekt macht allerdings die enthaltene suspendierte TS aus. Diese limitiert die Menge an frischem Mehl, die in der Anmaischung zugegeben werden kann. Ebenso erhöht sich die Viskosität, wodurch die Pumpfähigkeit beeinträchtigt wird. Als Alternative wird deshalb nach einer Temperaturbehandlung der Dünnschlempe (93°C - 177°C, 3 - 180 min) in einem weiteren Trennprozess eine wässrige Fraktion aus der Dünnschlempe abgetrennt, das sogenannte stick water, das weniger als 1% suspendierte TS enthält und besser für die Rückführung geeignet ist. Dieser Prozess ist jedoch apparativ sehr aufwändig und energieintensiv.

Teil des in EP2847341 A1 offenbarten Verfahrens ist eine Rückführung von Dünnschlempe (TS ca. 10%) oder Schlempe (TS ca. 16%) bis zu einem Anteil von 45% in die Anmaischung. Die Maische wird vor der Fermentation einem Jet-Cooking unterzogen, d.h. einem Hochtemperatur-Aufschluss (100°C - 150°C) unter Druck (3 bar - 5 bar), um die Stärke zu verflüssigen und die Viskosität zu reduzieren.

Als Nachteile sind hier die hohen Aufwendungen für die Apparate und der hohe Energiebedarf zu bewerten.

US 2015/0305370 A1 beschreibt ein Verfahren zur Steuerung des Proteingehaltes von Nebenprodukten eines Fermentationsprozesses, der wiederum Teil eines Prozesses zur Bioethanolgewinnung aus Getreide ist. Das Verfahren umfasst den Schritt des Auftrennens der nach Destillation der Getreidemaische erhaltenen Schlempe in Treber und Dünnschlempe. Dann werden proteinreiche Nebenprodukte aus der Dünnschlempe entfernt und es fällt eine proteinreduzierte Dünnschlempe an. Anschließend werden weitere Nicht-Protein-Komponenten aus der proteinreduzierten Dünnschlempe entfernt und der gesamte oder ein Teil der derart behandelten proteinreduzierten Dünnschlempe zu mindestens einem Teil des Trebers gegeben.

US 2019/0119711 A1 beschreibt ein Verfahren zur Herstellung eines hochkonzentrierten Sirups mittels eines Trockenmahlverfahrens. Das Verfahren beschreibt einen Trennprozess der Schlempe, wobei der grobe Feststoffanteil vermahlen wird und die Flüssigkeit einem Zentrifugationsprozess, unter Verwendung von Hochgeschwindigkeitszentrifugen, zugeführt und anschließend verdampft wird.

Derzeit sind keine Verfahren bekannt, bei denen eine gezielte Anreicherung der Proteine in der Maische und folglich in der Schlempe durch eine Rückführung der Schlempe in die Anmaischung erreicht wird. Durch eine Anreicherung der Proteine wird deren Abtrennung vereinfacht und die Wertigkeit des erzeugten Produktes aufgrund eines erhöhten Rohproteingehaltes gesteigert. Die Rückführung signifikanter Mengen an Schlempe ohne einen zusätzlichen energieintensiven Behandlungsschritt in einem wirtschaftlichen Produktionsprozess ist ebenso nicht beschrieben.

### ZUSAMMENFASSUNG DER ERFINDUNG

Es ist daher Aufgabe der Erfindung, die Proteine in einem effizienten energie- und ressourcenschonenden Fermentationsprozess gezielt anzureichern, einfach und kostengünstig abzutrennen und ein Proteinprodukt mit höherer Wertigkeit und verbesserter Marktfähigkeit zu erzeugen.

Die Aufgabe wird durch ein Verfahren zur Gewinnung einer konzentrierten proteinreichen Phase aus Reststoffen der Bioethanolproduktion gemäß Anspruch 1 gelöst, welches dadurch gekennzeichnet ist, dass:
a) vermahlenes Getreide vor der Fermentation in einem Kaltmaischverfahren angemaischt wird,
b) die fermentierte Maische einer Destillation zugeführt wird, bei der Schlempe als Reststoff entsteht,
c) die Schlempe aus b) einer Fest-Flüssig-Trennung zugeführt wird, wobei als Feststoff Treber und als flüssige Phase Dünnschlempe anfällt, und der anfallende Feststoff wird aus dem Prozess ausgeschleust wird,
d) die Dünnschlempe aus c) anteilig zurück in die Anmaischung geführt wird, wobei mindestens 0,15 kg Dünnschlempe-TS pro kg Getreide-TS zurückgeführt werden und durch das Rückführen der Dünnschlempe in die Anmaischung der Rohproteingehalt erhöht wird, wobei im gesamten Prozess ein pH< 4,0 eingehalten wird, und
e) mindestens ein Teil der flüssigen Phase aus c) Dünnschlempe mit einer wässrigen Prozessflüssigkeit verdünnt und einem weiteren Trennprozess zugeführt wird, wobei die dabei abgetrennte wässrige Phase, die Klarphase, vorwiegend die gelöste Trockensubstanz und die erzeugte konzentrierte Phase, das Konzentrat, vorwiegend die proteinreiche suspendierte Trockensubstanz enthält.

Die mit dieser Erfindung erzielten Vorteile bestehen insbesondere darin, dass durch die Prozessbedingungen (niedriger pH-Wert, geringe Temperaturen) die Proteine als kleine Partikeln in der Dünnschlempe vorliegen, diese durch die anteilige Rückführung der Dünnschlempe in die Anmaischung angereichert werden, wodurch sich der Rohproteingehalt signifikant erhöht und die Proteine ohne den Zusatz von Chemikalien und ohne einen energieintensiven Behandlungsschritt einfach abgetrennt werden können.

Vorteilhafte Weiterbildungen sind in den abhängigen Ansprüchen genannt. Der Wortlaut aller Ansprüche wird durch die Bezugnahme zum Inhalt der Beschreibung gemacht.

In einer bevorzugten Ausführungsform ist das erfindungsgemäße Verfahren so ausgestaltet, dass das Getreide einer Trockenvermahlung zugeführt wird.

In einer weiteren bevorzugten Ausführungsform ist das erfindungsgemäße Verfahren so ausgestaltet, dass die Anmaischung pro kg Getreide-TS bevorzugt mit mindestens 0,25 kg Dünnschlempe-TS und besonders bevorzugt mit mindestens 0,35 kg Dünnschlempe-TS erfolgt.

In einer weiteren bevorzugten Ausführungsform ist das erfindungsgemäße Verfahren so ausgestaltet, dass der Rohproteingehalt der Dünnschlempe durch die anteilige Rückführung in die Anmaischung im Vergleich zum Rohproteingehalt des Getreides abzüglich der Stärke um mindestens 5%rel. und bevorzugt um mindestens 10%rel. gesteigert wird.

In einer weiteren bevorzugten Ausführungsform ist das erfindungsgemäße Verfahren so ausgestaltet, dass durch die spezielle Fahrweise, d.h. das Rückführen signifikanter Dünnschlempemengen in die Anmaischung und damit das mehrfache Durchlaufen der Destille, der überwiegende Anteil an Partikeln in der Dünnschlempe eine Größe kleiner 50 µm aufweist.

In einer weiteren bevorzugten Ausführungsform ist das erfindungsgemäße Verfahren so ausgestaltet, dass die Dünnschlempe einen TS-Gehalt von mindestens 8% aufweist.

In einer weiteren bevorzugten Ausführungsform ist das erfindungsgemäße Verfahren so ausgestaltet, dass durch den gezielten Einsatz von Enzymen, z.B. Cellulasen, Hemicellulasen, Trehalase, Xylanase, Amylasen, Lipasen, Phytase und/oder Kombinationen daraus, nicht-proteinhaltige suspendierte Substanzen gespalten und in Lösung gebracht werden.

In einer weiteren bevorzugten Ausführungsform ist das erfindungsgemäße Verfahren so ausgestaltet, dass die Fest-Flüssig-Trennung der Schlempe in einer Filterpresse oder in einem Dekanter erfolgt.

In einer weiteren bevorzugten Ausführungsform ist das erfindungsgemäße Verfahren so ausgestaltet, dass die Trennkorngröße bei der Fest-Flüssig-Trennung der Schlempe im Bereich 150 - 500 µm, vorzugsweise 200 - 300 µm liegt.

In einer weiteren bevorzugten Ausführungsform ist das erfindungsgemäße Verfahren so ausgestaltet, dass die Dünnschlempe im gesamten Prozess einer maximalen Temperatur von 90°C ausgesetzt ist.

In einer weiteren bevorzugten Ausführungsform ist das erfindungsgemäße Verfahren so ausgestaltet, dass die Dünnschlempe vor dem Trennprozess einer enzymatischen Behandlung zur Spaltung nichtproteinhaltiger suspendierter Substanzen zugeführt werden kann.

In einer weiteren bevorzugten Ausführungsform ist das erfindungsgemäße Verfahren so ausgestaltet, dass die anteilige Verdünnung der Dünnschlempe mit einer geeigneten Prozessflüssigkeit, wie z.B. Kondensat aus der Dünnschlempe-Eindampfung, erfolgt.

In einer weiteren bevorzugten Ausführungsform ist das erfindungsgemäße Verfahren so ausgestaltet, dass die Dünnschlempe vor der Trennung in einem Verhältnis 1:5, vorzugsweise 1:3 und besonders bevorzugt 1:1 mit einer geeigneten Prozessflüssigkeit verdünnt wird.

In einer weiteren bevorzugten Ausführungsform ist das erfindungsgemäße Verfahren so ausgestaltet, dass der Zulauf vor dem Trennschritt einer Vorreinigung zugeführt wird.

In einer weiteren bevorzugten Ausführungsform ist das erfindungsgemäße Verfahren so ausgestaltet, dass die Vorreinigung des Zulaufs mithilfe eines Drehbürstensiebes oder eines Kantenspaltfilters erfolgt.

In einer weiteren bevorzugten Ausführungsform ist das erfindungsgemäße Verfahren so ausgestaltet, dass die Trennung der verdünnten Dünnschlempe in Klarphase und Konzentrat mithilfe eines Separators bei mindestens 2800xg, bevorzugt mindestens 3500xg und besonders bevorzugt mindestens 5000xg erfolgt.

In einer weiteren bevorzugten Ausführungsform ist das erfindungsgemäße Verfahren so ausgestaltet, dass das Konzentrat einen um mindestens 15%rel. gesteigerten Rohproteingehalt im Vergleich zur Dünnschlempe aufweist.

In einer weiteren bevorzugten Ausführungsform ist das erfindungsgemäße Verfahren so ausgestaltet, dass das Konzentrat einen Rohproteingehalt von mindestens 44%TS und bevorzugt mindestens 48%TS aufweist.

In einer weiteren bevorzugten Ausführungsform ist das erfindungsgemäße Verfahren so ausgestaltet, dass maximal 1/3 der TS im Konzentrat in gelöster Form vorliegen.

In einer weiteren bevorzugten Ausführungsform ist das erfindungsgemäße Verfahren so ausgestaltet, dass die Klarphase mindestens anteilig in den Prozess zurückgeführt wird, z.B. in die Dünnschlempe-Eindampfung.

In einer weiteren bevorzugten Ausführungsform ist das erfindungsgemäße Verfahren so ausgestaltet, dass die Klarphase zur Steigerung der Proteinausbeute mindestens anteilig einer Ultrafiltrationseinheit zugeführt werden kann.

In einer weiteren bevorzugten Ausführungsform ist das erfindungsgemäße Verfahren so ausgestaltet, dass das Konzentrat mindestens teilweise direkt als hochwertiges Proteinfuttermittel verwendet oder weiteren Behandlungsschritten zugeführt wird.

In einer weiteren bevorzugten Ausführungsform ist das erfindungsgemäße Verfahren so ausgestaltet, dass das Konzentrat zur Steigerung des Rohproteingehaltes einer enzymatischen Behandlung mit anschließender Fest-Flüssig-Trennung zugeführt werden kann.

Das erfindungsgemäße Verfahren betrifft insbesondere auch alle Kombinationen der oben beschriebenen, bevorzugten Ausführungsformen.

### Beschreibung der Lösung

Der Rohproteingehalt ist abhängig von der Getreideart. Er beträgt in den kleinkörnigen Getreiden, wie Weizen, Roggen, Triticale und Gerste ca. 10 - 15%TS und im Mais ca. 9%TS. Getreidekörner sind prinzipiell aus Schalen, Mehlkörper (Endosperm) und Keimling aufgebaut. Die in den Getreidekörnern enthaltenen Proteine befinden sich hauptsächlich im Mehlkörper, dieser weist etwa 70% der Proteine auf. Dies gilt sowohl für Weizen und Roggen als auch für Mais. Bei den kleinkörnigen Getreiden liegen ca. 10% der Proteine im Keimling vor, im Mais hingegen ca. 20%, da der Maiskeimling wesentlich größer ist. Die restlichen Proteine verteilen sich auf die übrigen Bestandteile.

Bei der Verarbeitung von Getreide zu Bioethanol werden die enthaltenen Proteine während der Fermentation durch die Hefe nicht abgebaut, sondern bleiben erhalten. Die vollständige Umsetzung der Stärke zu Ethanol würde eine Aufkonzentrierung des Rohproteingehaltes um ca. Faktor 3 im Vergleich zum Ausgangsmaterial bewirken. Darüber hinaus enthalten und produzieren die Hefezellen selbst auch Proteine. Am Ende der Fermentation bzw. nach der Abtrennung des produzierten Ethanols aus der Maische liegen in der Schlempe also Getreide- und Hefeproteine vor.

In der Regel weist Schlempe einen TS-Gehalt von mindestens 10% und einen Rohproteingehalt von mindestens ca. 25%TS auf. Der TS-Gehalt setzt sich aus suspendierten und gelösten Anteilen zusammen, die in etwa im gleichen Verhältnis vorliegen.

Dem Fachmann ist bekannt, dass die Schlempe zumindest anteilig einer Fest-Flüssig-Trennung unterzogen wird, um die Dünnschlempe einer Eindampfung oder Trocknung zuzuführen. Der abgetrennte Feststoff, häufig als Treber bezeichnet, wird aufgrund des hohen Fasergehaltes entweder unbehandelt oder getrocknet hauptsächlich als Futtermittel für Rinder eingesetzt.

Wünschenswert ist, aus der Schlempe in einem kostengünstigen, einfachen und effizienten Verfahren ein konkurrenzfähiges, proteinreiches, faserarmes Produkt zu erzeugen, das aufgrund des höheren Nährwertes eine höhere Wertschöpfung aufweist und eine breitere Anwendung findet.

Überraschenderweise wurde festgestellt, dass eine Rückführung signifikanter Dünnschlempemengen in Kombination mit dem Kaltmaischverfahren wirtschaftlich umgesetzt werden kann. Zum Anmaischen werden pro kg Getreide-TS mindestens 0,15 kg, bevorzugt mindestens 0,25 kg und besonders bevorzugt mindestens 0,35 kg Dünnschlempe-TS zurückgeführt. Aus dieser Fahrweise resultieren Ethanolgehalte in der fermentierten Maische im Bereich 5 - 8 Ma%. Bisher war bekannt, dass solch eine hohe Rückführung nur in Kombination mit einem Jet-Cooking umsetzbar ist.

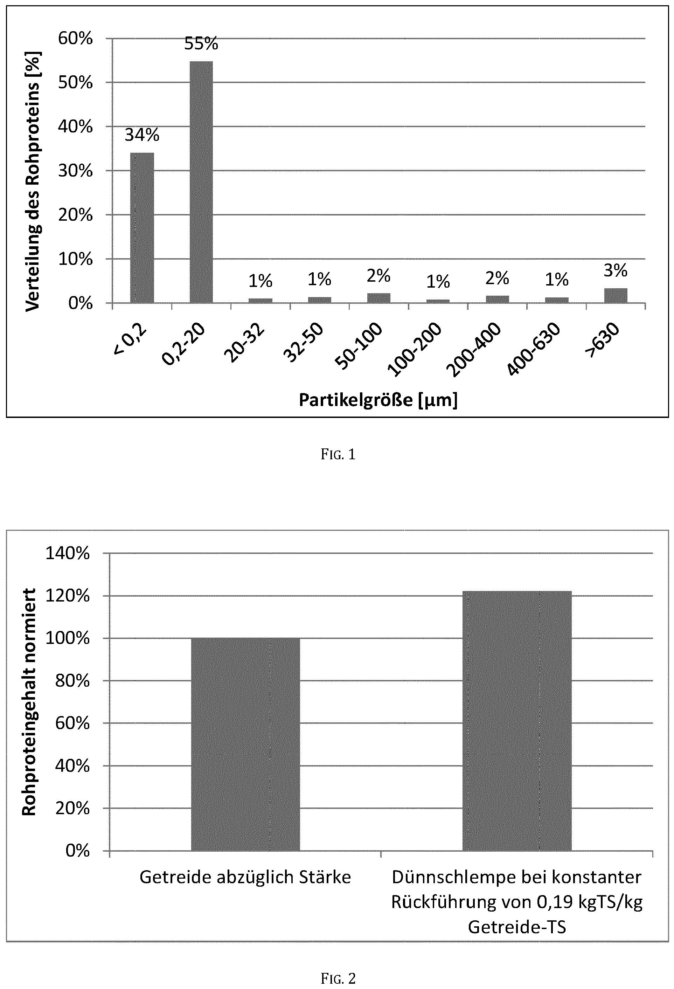

Weiterhin wurde festgestellt, dass das Hauptpotential zur Erzeugung eines proteinreichen Produktes in der Dünnschlempe liegt, da mehr als 70% des Rohproteins in der Schlempe in Partikeln kleiner als 50 µm enthalten sind und diese Partikeln bei der Fest-Flüssig-Trennung zu mindestens 70% in der Dünnschlempe verbleiben. In Fig. 1 ist die Verteilung des Rohproteins in den einzelnen Partikelgrößenfraktionen am Beispiel einer Schlempe aus dem vorliegenden Prozess dargestellt. In diesem Beispiel enthalten die Partikeln < 50 µm ca. 90% des Rohproteins. Der Anteil an Proteinen, die mit dem Treber abgetrennt werden, ist folglich gering.

Durch die Kombination aus Fest-Flüssig-Trennung und hoher Dünnschlempe-Rückführung reichern sich die proteinreichen Partikeln an, wodurch sich der Rohproteingehalt signifikant erhöht.

Zudem hat die hohe Rückführung eine erhebliche Reduktion des Bedarfs an Frischwasser zur Folge, wodurch der Prozess effizienter und ressourcenschonender wird. Der Frischwasserbedarf kann insgesamt auf ein Minimum reduziert oder gänzlich gedeckt werden, indem weitere Prozessflüssigkeiten, z.B. Kondensat aus der Dünnschlempe-Eindampfung oder Lutterwasser aus der Destillation, anteilig zurück in die Anmaischung geführt werden.

Ein Vergleich der Partikelgrößen der Schlempe aus dem vorliegenden Prozess mit denen einer Schlempe aus einer externen Bioethanolproduktionsanlage zeigt, dass die Partikeln in der Schlempe des beschriebenen Prozesses signifikant kleiner sind (Tab. 1). Im Durchschnitt sind diese Partikeln nur etwa halb so groß wie die in der externen Schlempe. Ebenso wird deutlich, dass die Dünnschlempe des vorliegenden Prozesses vorwiegend nur sehr kleine Partikeln enthält, da 50% der Partikeln ≤ 10 µm sind (D10). Diese kleinen Partikelgrößen sind ein deutlicher Anhaltspunkt dafür, dass die spezielle Fahrweise, d.h. die Rückführung signifikanter Dünnschlempemengen in die Anmaischung und damit das mehrfache Durchlaufen der Destille, die Partikelgrößen reduziert.

**Tab. 1**

| | **Schlempe extern** | **Schlempe** | **Dünnschlempe** |
|---|---|---|---|
| Durchschnittswert [µm] | 485,7 | 260,0 | 37,0 |
| D10 [µm] | 10,3 | 6,2 | 4,6 |
| D50 [µm] | 412,2 | 60,7 | 10 |
| D90 [µm] | 1188,8 | 783,3 | 119,3 |

Nachfolgend wird die Erfindung auf Grundlage der Gewinnung einer konzentrierten proteinreichen Phase aus Schlempen der Bioethanolproduktion erläutert.

Getreide, wie Roggen, Weizen, Triticale, Gerste, Mais und/oder Kombinationen daraus, wird trockenvermahlen und in einem effizienten energie- und ressourcenschonenden Fermentationsprozess zu Bioethanol verarbeitet. Das vermahlene Getreide wird mit einem Gemisch aus Prozessflüssigkeiten angemaischt. Für den Fall, dass die zur Verfügung stehenden Prozessflüssigkeiten nicht ausreichen und/oder Stoffe enthalten, die die Fermentation stören, z.B. Essigsäure in erhöhter Konzentration, wird zusätzlich Wasser in die Anmaischung gefahren. Der Aufschluss des eingemaischten Getreidemehls erfolgt nach dem Kaltmaischverfahren, d.h. unterhalb der Verkleisterungstemperatur der Stärke, vorzugsweise zwischen 45°C und 66°C. Diese geringen Temperaturen sind aufgrund des niedrigen Energiebedarfs besonders vorteilhaft.

Durch den Zusatz von Enzymen im Ethanol-Prozess, wie z.B. Cellulasen, Amylasen, Trehalase, Phytase, Hemicellulasen, Lipasen und/oder Kombinationen daraus, werden nicht-proteinhaltige Substanzen gespalten und in Lösung gebracht und somit von der suspendierten in die gelöste Fraktion überführt, die im weiteren Verlauf gezielt abgetrennt wird.

Bis zur Destillation wird eine Temperatur von 66°C nicht überschritten und im weiteren Prozessverlauf werden für eine begrenzte Zeit maximal 90°C erreicht. Der pH-Wert ist konstant < 4,0. Aufgrund des niedrigen pH-Werts, der geringen Temperaturen und der daraus resultierenden teilweisen Denaturierung, liegen die Proteine als kleine Partikeln vor, die bei der Fest-Flüssig-Trennung der Schlempe in der Dünnschlempe verbleiben, wodurch im Prozess eine gezielte Proteinanreicherung durch die anteilige Dünnschlempe-Rückführung möglich ist. Der niedrige pH-Wert bewirkt außerdem, dass kleine Proteine überwiegend ungelöst vorliegen und dadurch einfacher separiert werden können. Besonders vorteilhaft an diesem Verfahren, bedingt durch die niedrigen Temperaturen, ist der besonders geringe Energiebedarf des Prozesses.

Nach der Abtrennung des produzierten Ethanols aus der Maische wird die Schlempe mindestens anteilig einer Fest-Flüssig-Trennung zugeführt. Für diese Trennung wird z.B. ein Dekanter oder eine Filterpresse genutzt. Die Trennkorngröße bei diesem Trennprozess liegt im Bereich 150 - 500 µm, vorzugsweise 200 - 300 µm. Der abgetrennte Feststoff, der maximal 15% des gesamten Rohproteins enthält, wird ausgeschleust und als Futtermittel vorwiegend für Rinder eingesetzt.

Die erzeugte Dünnschlempe weist einen TS-Gehalt von mindestens 8% sowie einen Rohproteingehalt von mindestens 30%TS auf. Die Anteile an gelöster und suspendierter Trockensubstanz sind in etwa gleich. Mindestens 70% des gesamten Rohproteins der Dünnschlempe sind in den Partikeln < 50 µm und damit in der suspendierten TS enthalten.

Besonders vorteilhaft ist es, die Anmaischung mit einem signifikanten Dünnschlempe-Anteil, d.h. mindestens 0,15 kg, bevorzugt mindestens 0,25 kg und besonders bevorzugt mindestens 0,35 kg Dünnschlempe-TS pro kg Getreide-TS durchzuführen, wodurch eine Anreicherung der kleinen, proteinreichen Partikeln erfolgt. Nach mehreren Umläufen mit der beschriebenen Dünnschlempe-Rückführung erreicht die Konzentration an diesen Partikeln einen konstanten Wert. Der Rohproteingehalt der Dünnschlempe erhöht sich dabei im Vergleich zum Getreide abzüglich der Stärke, d.h. im Vergleich zum Rohproteingehalt, der sich nach vollständiger Umsetzung der Stärke ergeben würde, signifikant um mindestens 5%rel. und wird bei gleichbleibender Rückführung konstant auf diesem Niveau gehalten.

Fig. 2 zeigt ein Beispiel, bei dem bei einer konstanten Rückführung von 0,19 kg Dünnschlempe-TS pro kg Getreide-TS in die Anmaischung der Rohproteingehalt in der Dünnschlempe im Vergleich zum Rohproteingehalt im Getreide abzüglich der Stärke um ca. 20%rel. gesteigert wird.

Bevor die Dünnschlempe einer Trennung unterzogen wird, kann eine enzymatische Behandlung mindestens eines Teils der Dünnschlempe mit Cellulasen, Hemicellulasen, Lipasen und/oder Amylasen bzw. Kombinationen daraus erfolgen, um ungelöste nicht-proteinhaltige Substanzen zu spalten und in die gelöste Fraktion zu überführen, wodurch der Rohproteingehalt der suspendierten TS zusätzlich gesteigert wird.

In einem besonders effizienten Prozess läuft die enzymatische Behandlung direkt unter den in der Dünnschlempe vorliegenden Bedingungen (Temperatur, pH) ab. Alternativ dazu kann der pH-Wert der Dünnschlempe durch Zugabe von Lauge, wie z.B. NaOH, KOH oder NH₄HCO₃, auf einen für die Enzyme optimalen Wert eingestellt werden, um eine optimale Enzymaktivität zu gewährleisten. Ebenso kann auch die Temperatur an die Erfordernisse der Enzyme angepasst werden. Die gleichzeitige Anpassung von pH und Temperatur ist vorteilhaft.

Zur Gewinnung einer proteinreichen Phase aus mindestens einem Teil der Dünnschlempe ist es von Vorteil, diese vor dem Trennschritt mit einer wässrigen Prozessflüssigkeit zu verdünnen. Dadurch wird zum einen eine Verringerung der Viskosität erzielt. Zum anderen bewirkt die Verdünnung eine Reduktion des Gehaltes an gelöster TS und damit des Salzgehaltes, wie z.B. Sulfat. Am geeignetsten ist eine wässrige Prozessflüssigkeit, die nahezu keine gelöste TS enthält.

Eine solche wässrige Prozessflüssigkeit entstammt z.B. der Dünnschlempe-Eindampfung, bei der aus mindestens einem Teil der anfallenden Dünnschlempe ein Dünnschlempe-Konzentrat erzeugt wird, das in etwa einen doppelt so hohen TS-Gehalt wie das Ausgangsmaterial aufweist.

Besonders vorteilhaft ist diese Prozessflüssigkeit, weil sie eine ähnliche Temperatur wie die Dünnschlempe selbst aufweist, sodass sich bei der Verdünnung eine Temperatur von ca. 50°C einstellt, die sich ebenfalls positiv auf die Viskosität und damit auf die nachfolgende Trennung auswirkt. Die Prozessflüssigkeiten können also mit ihrer anfallenden Temperatur energetisch effizient genutzt werden, ohne dass eine Temperierung erforderlich ist.

Die Dünnschlempe sollte vorzugsweise in einem Verhältnis 1:5, bevorzugt 1:3 und besonders bevorzugt 1:1 verdünnt werden. Grundsätzlich gilt je stärker die Dünnschlempe verdünnt wird, umso einfacher können die suspendierten proteinreichen Partikeln im folgenden Trennprozess abgeschieden werden. Der Anteil an Dünnschlempe muss jedoch so groß sein, dass signifikante Mengen an suspendierter TS abtrennbar sind. Die Verdünnung sollte also so gewählt werden, dass der Prozess ökonomisch sinnvoll umsetzbar ist.

Zur Gewährleistung einer optimalen Trennung sollte die verdünnte Dünnschlempe, Zulauf genannt, einer Vorreinigung unterzogen werden, um eventuell noch enthaltene grobe Partikeln und Fremdstoffe abzuscheiden, die die Trennung beeinträchtigen bzw. das Proteinprodukt verunreinigen würden. Die Vorreinigung kann z.B. in Form einer Fest-Flüssig-Trennung mithilfe eines groben Filters erfolgen.

Für die Trennung des Zulaufs kommt ein Separator zum Einsatz. In diesem Schritt reichert sich die suspendierte TS in der konzentrierten Phase, als Konzentrat bezeichnet, an, wohingegen die gelöste TS vorwiegend über die wässrige Phase ausgetragen wird, die sogenannte Klarphase.

Eine Möglichkeit zur Optimierung der Trennung besteht in der Temperierung des Zulauf-Stroms auf Temperaturen im Bereich 50°C - 90°C, wodurch die Viskosität reduziert und eine kleinere Trennkorngröße erzielt werden kann. Je kleiner die Trennkorngröße, umso weniger proteinreiche Partikeln werden mit der Klarphase abgeschieden. Ob eine Temperierung >50°C sinnvoll ist, muss anhand der Proteinausbeute und des notwendigen Energiebedarfs bewertet werden.

Eine mehrstufige Trennung ist ebenso möglich und vorteilhaft, um den Rohproteingehalt zu steigern und den Salzgehalt zu reduzieren. Dazu wird das Konzentrat nach dem ersten Trennschritt wieder mit einer wässrigen Prozessflüssigkeit, z.B. Kondensat aus der Dünnschlempe-Eindampfung, verdünnt und einer erneuten Trennstufe, z.B. einem Separator, zugeführt.

Optional kann das Konzentrat vor dieser Trennstufe mit Enzymen, z.B. Cellulasen, Hemicellulasen, Lipasen und/oder Amylasen bzw. Kombinationen daraus, behandelt werden, um ungelöste nicht-proteinhaltige Substanzen zu spalten und in die gelöste Fraktion zu überführen. Eine enzymatische Behandlung an dieser Stelle im Prozess ist besonders vorteilhaft, da aufgrund der geringeren Mengenströme weniger Enzym erforderlich ist und ggf. notwendige Verweilzeitbehälter kleiner dimensioniert werden können.

In der Klarphase macht der Anteil an gelöster TS mindestens 2/3 der Gesamt-TS aus. Das Konzentrat weist einen Anteil an gelöster TS von maximal 1/3 der Gesamt-TS auf und der Rohproteingehalt ist im Vergleich zur Dünnschlempe um mindestens 15%rel. erhöht.

Zur Steigerung der Effizienz des gesamten Prozesses und der Proteinausbeute ist es von Vorteil, die Klarphase mindestens anteilig einer Ultrafiltrationseinheit zuzuführen, um noch enthaltene Proteine zu gewinnen. Das daraus entstehende Permeat kann in die Dünnschlempe-Eindampfung zurückgeführt werden.

Alternativ kann die Klarphase auch einer Eindampfung unterzogen bzw. der Dünnschlempe-Eindampfung beigemischt werden. Das entstehende Kondensat kann zurück in die Anmaischung geführt und/oder zur Verdünnung der Dünnschlempe bzw. des Konzentrats bei mehrstufiger Trennung genutzt werden. Eine direkte Rückführung der Klarphase in die Anmaischung ist nicht ratsam, um eine Anreichung des nicht-proteinhaltigen gelösten TS zu vermeiden.

Das proteinreiche Konzentrat kann sowohl direkt als hochwertiges Proteinfuttermittel verwendet als auch nachfolgenden Behandlungsschritten zur weiteren Aufkonzentrierung zugeführt werden, wie z.B. einer Eindampfung oder einer Sprühtrocknung.

### KURZBESCHREIBUNG DER ZEICHNUNGSFIGUREN

- FIG. 1: ZEIGT DIE VERTEILUNG DES ROHPROTEINS IN DEN EINZELNEN PARTIKELGRÖßENFRAKTIONEN AM BEISPIEL EINER SCHLEMPE AUS DEM VORLIEGENDEN PROZESS
- FIG. 2: ZEIGT EIN BEISPIEL FÜR NORMIERTE ROHPROTEINGEHALTE VON GETREIDE ABZÜGLICH DER STÄRKE UND VON EINER DÜNNSCHLEMPE BEI EINER KONSTANTEN RÜCKFÜHRUNG VON 0,19 KG DÜNNSCHLEMPE-TS/KG GETREIDE-TS (NORMIERT AUF ROHPROTEINGEHALT GETREIDE ABZGL. STÄRKE)
- FIG. 3: ZEIGT EIN FLIEßSCHEMA EINER MÖGLICHEN AUSFÜHRUNGSFORM GEMÄß DER ERFINDUNG
- FIG. 4: ZEIGT EIN FLIEßSCHEMA EINER MÖGLICHEN AUSFÜHRUNGSFORM GEMÄß DER ERFINDUNG IN FORM EINER ZWEISTUFIGEN TRENNUNG

### DETAILLIERTE BESCHREIBUNG BEVORZUGTER AUSFÜHRUNGSFORMEN

### AUSFÜHRUNGSBEISPIEL 1

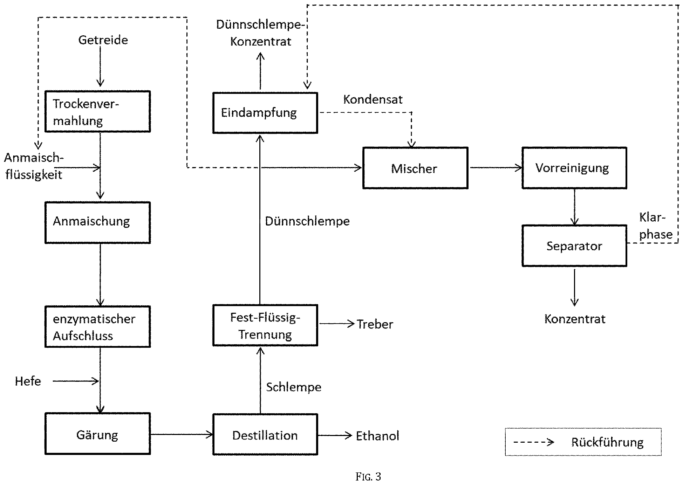

Die Möglichkeit einer technischen Ausführung des Verfahrens sei im Folgenden an der Gewinnung einer konzentrierten proteinreichen Phase aus Triticale-Roggen-Dünnschlempe veranschaulicht (Fig. 3).

Eine Triticale-Roggen-Mischung enthält folgende Inhaltsstoffe:

**Tab. 2**

| | |
|---|---|
| Trockensubstanz | 85%OS |
| Stärke | 67%TS |
| Rohprotein | 11%TS |

| | |
|---|---|
| OS - Originalsubstanz | |

Bei vollständiger Umsetzung der Stärke würde sich ein Rohproteingehalt von 34%TS ergeben.

Vor der Fermentation wird eine Triticale-Roggen-Mischung trockenvermahlen und mit 0,24 kg Dünnschlempe-TS pro kg Getreide-TS und Wasser im Verhältnis 1:4,0, d.h. ein Teil Getreide und 4 Teile Flüssigkeit, angemaischt. Der enzymatische Aufschluss der Maische erfolgt bei ca. 47°C. Durch Zugabe der Hefe wird die Vergärung gestartet, die in etwa 60 h dauert. Aus der fermentierten Maische wird in der Destillation anschließend das produzierte Ethanol abgetrennt.

Die entstandene Schlempe wird mit einem Massenstrom von ca. 71 t/h einer Fest-Flüssig-Trennung in einem Dekanter zugeführt. Die Trennkorngröße des Dekanters beträgt ca. 250 µm. Alle Partikeln, die größer als 390 µm sind, werden in diesem Schritt vollständig abgetrennt und enthalten 5% des ursprünglich in der Schlempe vorhandenen Rohproteins. Etwa 76% der proteinreichen Partikeln < 50 µm verbleiben in der Dünnschlempe. Der Massenstrom des abgetrennten Feststoffs, der ausgeschleust wird, beträgt ca. 7 t/h, der Dünnschlempe-Massenstrom ca. 64 t/h.

Die Dünnschlempe weist einen TS-Gehalt von ca. 13% auf, wovon jeweils etwa die Hälfte in suspendierter bzw. gelöster Form vorliegt. Die durchschnittliche Partikelgröße beträgt 38 µm, 3/4 der Partikeln sind kleiner als 20 µm. Die Partikeln < 20 µm enthalten ca. 90% des Rohproteins.

Ein Dünnschlempe-Massenstrom von 31 t/h wird zurück in die Anmaischung geführt (in Fig. 3 gestrichelt dargestellt), 19 t/h gehen in die Eindampfung. Die restliche Dünnschlempe wird in einen Sammelbehälter geleitet und von dort aus in einen gerührten Behälter, in den gleichzeitig das Kondensat aus der Dünnschlempe-Eindampfung geführt wird (in Fig. 3 gestrichelt dargestellt). Die Dünnschlempe und das Kondensat werden im Verhältnis 1:1 gemischt.

Die verdünnte Dünnschlempe, im weiteren Verlauf als Zulauf bezeichnet, weist eine Temperatur von 50°C auf und wird zunächst in einem Drehbürstensieb vorgereinigt, um grobe Partikeln und Verunreinigungen abzuscheiden. Die Spaltbreite des Filtersiebes beträgt 0,4 mm.

Mit einem Massenstrom von 12 t/h wird der vorgereinigte Zulauf zum Düsenseparator geführt. Dieser enthält 12 Düsen, die an der Unterseite der Trommel angeordnet sind und einen Durchmesser von 0,7 mm aufweisen. Die Trennung des Zulaufs im Separator erfolgt bei ca. 5500xg, wobei die Klarphase über den Greifer im oberen Teil herausströmt. Der Feststoff sammelt sich am Rand und wird durch die Düsen an der Unterseite ausgetragen, die den Konzentratstrom begrenzen und so für eine Anreicherung sorgen.

In Tabelle 3 sind die Massenströme, TS- und Rohproteingehalte der einzelnen Ströme des Düsenseparators gegenübergestellt. Der Rohproteingehalt des Konzentrats verdeutlicht, dass die proteinreichen Partikeln unter den genannten Prozessbedingungen erfolgreich angereichert werden konnten. Die Ausbeute an Rohprotein beträgt in diesem Beispiel 47%.

**Tab. 3**

| | | Zulauf | Klarphase | Konzentrat |
|---|---|---|---|---|
| Massenstrom | [t/h] | 12,0 | 9,8 | 2,2 |
| Trockensubstanzgehalt | [Ma%] | 6,2 | 4,6 | 13,3 |
| Rohproteingehalt | [%TS] | 41 | 36 | 50 |

Die abgetrennte Klarphase wird in die Eindampfung zurückgeführt (in Fig. 3 gestrichelt dargestellt) und das entstehende Kondensat steht wiederum als Verdünnungsmedium für die Dünnschlempe zur Verfügung. Das erzeugte proteinreiche Konzentrat wird gesammelt und entweder direkt oder nach einer Eindampfung oder Sprühtrocknung als hochwertiges Proteinfuttermittel verwendet.

Im Vergleich zu gegenwärtig auf dem Markt verfügbaren Futtermitteln, die aus Reststoffen einer Bioethanolproduktion erzeugt wurden (Tab. 4), weist das Konzentrat aufgrund des signifikant gesteigerten Rohproteingehaltes eine deutlich höhere Wertigkeit auf.

**Tab. 4**

| | TS [%TS] | Rohproteingehalt [%TS] |
|---|---|---|
| ProtiGrain ^{®(1)} | 90 | 29 |
| Weizen-/Gerstetrockenschlempe⁽²⁾ | 90 | 38 |
| Pressschlempe⁽³⁾ | 34,5 | 27 |
| GrainPro⁽⁴⁾ | 27 | 37 |

| | | |
|---|---|---|
| (1) HTTP://WWW.CROPENERGIES.COM/DE/DOWNLOADS/BROSCHUEREN/BROSCHUEREN-DATEIEN/DATENBLATT_PROTIGRAIN/DATENBLATTPROTIGRAIN_JULI_2016.PDf (ABGERUFEN 13.03.19) (2) HTTPS://WWW.LFL.BAYERN.DE/MAM/CMS07/ITE/DATEIEN/TROCKENSCHLEMPE_MERKBLATT.PDF (ABGERUFEN AM 13.03.19) (3) HTTP://FUTTERMITTEL-GETREIDETECHNIK.DE/PAGES/PRODUKTE/PRESS-SCHLEMPE.PHP (ABGERUFEN AM 13.03.19) (4) HTTP://FUTTERMITTEL-GETREIDETECHNIK.DE/PAGES/PRODUKTE/PRESS-SCHLEMPE.PHP (ABGERUFEN AM 13.03.19) | | |

### AUSFÜHRUNGSBEISPIEL 2

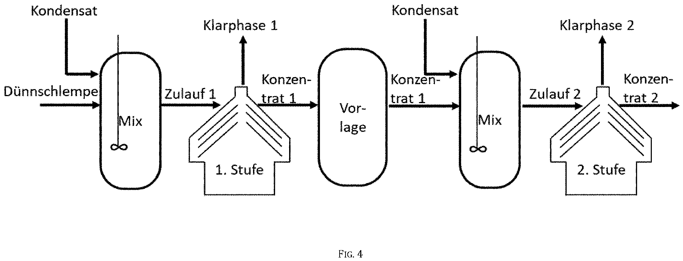

Eine weitere Möglichkeit einer technischen Ausführung des Verfahrens sei im Folgenden an der Gewinnung einer konzentrierten proteinreichen Phase durch eine zweistufige Trennung in einem Düsenseparator veranschaulicht (Fig. 4).

Dünnschlempe aus dem Ethanolprozess wird in einem gerührten Behälter mit Kondensat aus der Dünnschlempe-Eindampfung im Verhältnis 1:1 gemischt. Dieser als Zulauf 1 bezeichnete Strom wird mit einem Massenstrom von 10,8 t/h zum Düsenseparator geführt und bei ca. 5500xg in 8,4 t/h Klarphase 1 sowie 2,4 t/h Konzentrat 1 getrennt.

Die relevanten Messgrößen der Ströme der 1. Trennstufe sind in Tabelle 5 gelistet. Die Ausbeute an Rohprotein beträgt 50%.

**Tab. 5**

| | | Zulauf 1 | Klarphase 1 | Konzentrat 1 |
|---|---|---|---|---|
| Massenstrom | [t/h] | 10,8 | 8,4 | 2,4 |
| Trockensubstanzgehalt | [Ma%] | 8,0 | 6,2 | 14,3 |
| Rohproteingehalt | [%TS] | 41 | 34 | 52 |

Das Konzentrat 1 wird zunächst in einem Vorlagebehälter gesammelt, da für den 2. Trennschritt ein Mindestzulauf zum Düsenseparator erforderlich ist. Wenn der Behälter gefüllt ist, wird das Konzentrat 1 in einen gerührten Behälter geleitet, in dem die Verdünnung mit Kondensat aus der Dünnschlempe-Eindampfung im Verhältnis 1:1 erfolgt. Dieser als Zulauf 2 bezeichnete Strom wird mit einem Massenstrom von 10,8 t/h zum Düsenseparator geführt und bei ca. 5500xg in 8,4 t/h Klarphase 2 und 2,4 t/h Konzentrat 2 getrennt.

Die relevanten Messgrößen der Ströme der 2. Trennstufe sind in Tabelle 6 gelistet. Die Ausbeute an Rohprotein in der 2. Trennstufe beträgt 62%.

**Tab. 6**

| | | Zulauf 2 | Klarphase 2 | Konzentrat 2 |
|---|---|---|---|---|
| Massenstrom | [t/h] | 10,8 | 8,4 | 2,4 |
| Trockensubstanzgehalt | [Ma%] | 6,8 | 3,7 | 17,4 |
| Rohproteingehalt | [%TS] | 53 | 47 | 58 |

Der Rohproteingehalt im Konzentrat 2 verdeutlicht, dass eine zweistufige Trennung eine signifikante Verbesserung des Proteinproduktes bewirkt. Die Gesamtausbeute an Rohprotein durch eine zweistufige Trennung beträgt in diesem Beispiel 31%.

### AUSFÜHRUNGSBEISPIEL 3

Eine weitere Möglichkeit einer Ausführung des Verfahrens im Labormaßstab sei im Folgenden an der Gewinnung einer konzentrierten proteinreichen Phase ausgehend von einem Konzentrat, das entsprechend Bsp. 1 erzeugt und anschließend enzymatisch behandelt wurde, veranschaulicht.

Das verwendete Konzentrat weist einen TS-Gehalt von 13,7% und einen Rohproteingehalt von 48%TS auf.

Für die enzymatische Behandlung werden 250g des Konzentrats in einen 500ml-Gärkolben eingewogen. Als Enzym wird ein Präparat mit Cellulase-, Hemicellulase- und Xylanase-Aktivität in einer Dosierung von 0,1%TS verwendet. Im Referenzansatz wird kein Enzym zum Konzentrat gegeben. Die Gärkolben werden mit einem Gärröhrchen verschlossen und in einem Schüttler bei 150 rpm und 33°C über 72h inkubiert.

Nach Ablauf der Inkubationszeit werden jeweils 100g des behandelten Materials (aus dem Kolben mit Enzym bzw. aus dem Kolben ohne Enzym) mit 100g Leitungswasser, das zuvor auf 33°C temperiert wurde, gemischt und anschließend bei 4770xg 15 min zentrifugiert. Der Rohproteingehalt beider Pellets wird bestimmt.

Zum Vergleich wird das Konzentrat, das als Ausgangsmaterial für die Versuche diente, auf 33°C temperiert und ebenso wie oben beschrieben mit Leitungswasser im Verhältnis 1:1 verdünnt und 15min bei 4770xg zentrifugiert. Das entstandene Pellet wird analysiert.

Die TS- und Rohproteingehalte der Pellets sind in Tabelle 7 zusammengefasst.

**Tab. 7**

| | | Pellet aus Konzentrat | Pellet aus Behandlung ohne Enzym | Pellet aus Behandlung mit Enzym |
|---|---|---|---|---|
| Trockensubstanzgehalt | [Ma%] | 25,0 | 25,2 | 24,4 |
| Rohproteingehalt | [%TS] | 56 | 56 | 61 |

Durch die enzymatische Behandlung wird der Rohproteingehalt im Pellet signifikant gesteigert. Der TS-Gehalt sinkt, da durch die Enzyme ungelöste nicht-proteinhaltige Substanzen gespalten und in Lösung gebracht werden, die nach der Zentrifugation im Überstand verbleiben.

## Patentansprüche

1. Verfahren zur Gewinnung einer konzentrierten proteinreichen Phase aus Reststoffen der Bioethanolproduktion, **gekennzeichnet dadurch, dass**
a) vermahlenes Getreide vor der Fermentation in einem Kaltmaischverfahren angemaischt wird,
b) die fermentierte Maische einer Destillation zugeführt wird, bei der Schlempe als Reststoff entsteht,
c) die Schlempe aus b) einer Fest-Flüssig-Trennung zugeführt wird, wobei als Feststoff Treber und als flüssige Phase Dünnschlempe anfällt, und der anfallende Feststoff aus dem Prozess ausgeschleust wird,
d) die Dünnschlempe aus c) anteilig zurück in die Anmaischung geführt wird, wobei mindestens 0,15 kg Dünnschlempe-TS pro kg Getreide-TS zurückgeführt werden und durch das Rückführen der Dünnschlempe in die Anmaischung der Rohproteingehalt erhöht wird, wobei im gesamten Prozess ein pH-Wert < 4,0 eingehalten wird, und
e) mindestens ein Teil der Dünnschlempe mit einer wässrigen Prozessflüssigkeit verdünnt und einem weiteren Trennprozess zugeführt wird, wobei die dabei abgetrennte wässrige Phase, die Klarphase, vorwiegend die gelöste Trockensubstanz und die erzeugte konzentrierte Phase, das Konzentrat, vorwiegend die proteinreiche suspendierte Trockensubstanz enthält.

2. Verfahren nach Anspruch 1 **gekennzeichnet dadurch, dass** die Anmaischung pro kg Getreide-TS bevorzugt mit mindestens 0,25 kg Dünnschlempe-TS und besonders bevorzugt mit mindestens 0,35 kg Dünnschlempe-TS erfolgt.

3. Verfahren nach einem der vorherigen Ansprüche **gekennzeichnet dadurch, dass** die Fest-Flüssig-Trennung der Schlempe in einer Filterpresse oder in einem Dekanter erfolgt.

4. Verfahren nach einem der vorherigen Ansprüche **gekennzeichnet dadurch, dass** der Rohproteingehalt der Dünnschlempe durch die anteilige Rückführung in die Anmaischung im Vergleich zum Rohproteingehalt des Getreides abzüglich der Stärke um mindestens 5%rel. und bevorzugt um mindestens 10%rel. gesteigert wird.

5. Verfahren nach einem der vorherigen Ansprüche **gekennzeichnet dadurch, dass** die anteilige Verdünnung der Dünnschlempe mit einer geeigneten Prozessflüssigkeit, wie z.B. Kondensat aus der Dünnschlempe-Eindampfung, erfolgt.

6. Verfahren nach einem der vorherigen Ansprüche **gekennzeichnet dadurch, dass** die Dünnschlempe vor der Trennung in einem Verhältnis 1:5, bevorzugt 1:3 und besonders bevorzugt 1:1 mit einer geeigneten Prozessflüssigkeit verdünnt wird.

7. Verfahren nach einem der vorherigen Ansprüche **gekennzeichnet dadurch, dass** die Trennung der verdünnten Dünnschlempe in Klarphase und Konzentrat mithilfe eines Separators bei mindestens 2800xg, bevorzugt mindestens 3500xg und besonders bevorzugt mindestens 5000xg erfolgt.

8. Verfahren nach einem der vorherigen Ansprüche **gekennzeichnet dadurch, dass** durch den gezielten Einsatz von Enzymen, z.B. Cellulasen, Hemicellulasen, Trehalase, Xylanase, Amylasen, Lipasen, Phytase und/oder Kombinationen daraus, nicht-proteinhaltige suspendierte Substanzen gespalten und in Lösung gebracht werden.

9. Verfahren nach einem der vorherigen Ansprüche **gekennzeichnet dadurch, dass** das Konzentrat einen um mindestens 15%rel. gesteigerten Rohproteingehalt im Vergleich zur Dünnschlempe aufweist.

10. Verfahren nach einem der vorherigen Ansprüche **gekennzeichnet dadurch, dass** das Konzentrat einen Rohproteingehalt von mindestens 44%TS und bevorzugt mindestens 48%TS aufweist.

11. Verfahren nach einem der vorherigen Ansprüche **gekennzeichnet dadurch, dass** die Klarphase mindestens anteilig in den Prozess zurückgeführt wird, z.B. in die Dünnschlempe-Eindampfung.

12. Verfahren nach einem der vorherigen Ansprüche **gekennzeichnet dadurch, dass** das Konzentrat mindestens teilweise direkt als hochwertiges Proteinfuttermittel verwendet oder weiteren Behandlungsschritten zugeführt wird.

## Claims

1. A method for obtaining a concentrated protein-rich phase from waste products of bioethanol production, **characterised in that**:
a) a ground cereal is mashed in a cold mashing process before fermentation;
b) the fermented mash is fed to a distillation process, which produces thick stillage as waste product;
c) the thick stillage from b) is fed to a solid-liquid separation, wherein spent grains are produced as the solid and thin stillage is produced as the liquid phase, and the resulting solid is discharged from the method;
d) the thin stillage is partially returned to the mashing process, with at least 0.15 kg thin stillage DM per kg of cereal DM being returned and the raw protein content of the thin stillage being increased by recirculating the thin stillage to the mashing process, wherein a pH value < 4.0 is maintained throughout the method; and
e) at least part of the thin stillage is diluted with an aqueous process liquid and fed to a further separation process, wherein the aqueous phase separated in this further separation process, the clear phase, containing predominantly the dissolved dry matter, and wherein the concentrated protein-rich phase produced in this further separation process, the concentrate, containing predominantly protein-rich suspended dry matter.

2. The method according to claim 1, **characterised in that** the mashing process is carried out with at least 0.25 kg thin stillage DM per kg of cereal DM and particularly preferably with at least 0.35 kg thin stillage DM per kg of cereal DM.

3. The method according to any one of the preceding claims, **characterised in that** the solid-liquid separation of the thick stillage takes place in a filter press or in a decanter.

4. The method according to any one of the preceding claims, **characterised in that**, by the partial recirculation to the mashing process, the raw protein content of the thin stillage is increased by at least 5%rel. and preferably by at least 10%rel. compared to the raw protein content of the cereal less starch.

5. The method according to any one of the preceding claims, **characterised in that** the partial dilution of the thin stillage is carried out with a suitable process liquid, such as condensate from the thin stillage evaporation.

6. The method according to any one of the preceding claims, **characterised in that** the thin stillage is diluted with a suitable process liquid in a ratio of 1:5, preferably 1:3 and particularly preferably 1:1 before separation.

7. The method according to any one of the preceding claims, **characterised in that** the further separation of the diluted thin stillage into the clear phase and the concentrate is performed with the aid of a separator and takes place at least at 2800xg, preferably at least 3500xg and particularly preferably at least 5000xg.

8. The method according to any one of the preceding claims, **characterised in that**, by a targeted use of enzymes selected from the group of cellulases, hemicellulases, trehalases, xylanases, amylases, lipases, phytases and/or combinations thereof, non-protein-containing suspended substances are cleaved and dissolved.

9. The method according to any one of the preceding claims, **characterised in that** the concentrate has a raw protein content increased by at least 15%rel. compared to the thin stillage.

10. The method according to any one of the preceding claims, **characterised in that** the concentrate has a raw protein content of at least 44%DM and preferably at least 48%DM.

11. The method according to any one of the preceding claims, **characterised in that** the clear phase is at least partially returned to the process, for example to the thin stillage evaporation.

12. The method according to any one of the preceding claims, **characterised in that** the concentrate is at least partially used directly as a high-quality protein feed or is fed to further treatment steps.

## Revendications

1. Procédé d'obtention d'une phase concentrée riche en protéines à partir de résidus de la production de bioéthanol, **caractérisé en ce que**
a) des céréales broyées sont brassées à froid avant la fermentation dans un procédé de brassage à froid,
b) le moût fermenté est soumis à une distillation qui produit des drêches comme résidu,
c) les drêches issues de b) sont soumises à une séparation solide-liquide, la matière solide obtenue étant des drêches et, en tant que phase liquide, des drêches minces, et la matière solide obtenue est évacuée du processus,
d) les drêches minces de c) sont recyclées proportionnellement dans le brassage, au moins 0,15 kg de MS de drêches minces étant recyclé par kg de MS de céréales et la teneur en protéines brutes étant augmentée par le recyclage des drêches minces dans le brassage, un pH < 4,0 étant maintenu tout au long du processus, et
e) au moins une partie des drêches minces est diluée avec un liquide de processus aqueux et soumis à un autre processus de séparation, la phase aqueuse ainsi séparée, la phase claire, contenant principalement la matière sèche dissoute, et la phase concentrée produite, le concentré, contenant principalement la matière sèche en suspension riche en protéines.

2. Procédé selon la revendication 1, **caractérisé en ce que** le brassage par kg de MS de céréales est effectué de préférence avec au moins 0,25 kg de MS de drêches minces et de manière particulièrement préférée avec au moins 0,35 kg de MS de drêches minces.

3. Procédé selon l'une quelconque des revendications précédentes, **caractérisé en ce que** la séparation solide-liquide des drêches est réalisée dans un filtre-presse ou dans un décanteur.

4. Procédé selon l'une quelconque des revendications précédentes, **caractérisé en ce que** la teneur en protéines brutes des drêches minces est augmentée d'au moins 5%rel, et de préférence d'au moins 10%rel, par le recyclage proportionnel dans le brassage, par rapport à la teneur en protéines brutes de la céréale diminuée de l'amidon.

5. Procédé selon l'une quelconque des revendications précédentes, **caractérisé en ce que** la dilution proportionnelle des drêches minces est effectuée par un liquide de processus approprié, tel qu'un condensat provenant de l'évaporation des drêches minces.

6. Procédé selon l'une des revendications précédentes, **caractérisé en ce que** les drêches minces sont diluées avant la séparation dans un rapport de 1:5, de préférence de 1:3 et de manière particulièrement préférée de 1:1 par un liquide de processus approprié.

7. Procédé selon l'une des revendications précédentes, **caractérisé en ce que** la séparation des drêches minces diluées en phase claire et en concentré est réalisée à l'aide d'un séparateur à au moins 2800xg, de préférence au moins 3500xg et de manière particulièrement préférée au moins 5000xg.

8. Procédé selon l'une des revendications précédentes, **caractérisé en ce que** des substances en suspension ne contenant pas de protéines sont décomposées et mises en solution par l'utilisation ciblée d'enzymes, par exemple cellulases, hémicellulases, tréhalase, xylanase, amylases, lipases, phytase et/ou des combinaisons de celles-ci.

9. Procédé selon l'une des revendications précédentes, **caractérisé en ce que** le concentré présente une teneur en protéines brutes augmentée d'au moins 15%rel par rapport aux drêches minces.

10. Procédé selon l'une des revendications précédentes, **caractérisé en ce que** le concentré présente une teneur en protéines brutes d'au moins 44%MS et de préférence d'au moins 48%MS.

11. Procédé selon l'une des revendications précédentes, **caractérisé en ce que** la phase claire est recyclée au moins proportionnellement dans le processus, par exemple dans l'évaporation des drêches minces.

12. Procédé selon l'une des revendications précédentes, **caractérisé en ce que** le concentré est au moins en partie utilisé directement comme aliment pour animaux protéique de haute qualité ou est soumis à d'autres étapes de traitement.
